# EUROPÄISCHE PATENTSCHRIFT

(11) **EP 4 025 161 B1**
(45) Veröffentlichungstag und Bekanntmachung des Hinweises auf die Patenterteilung: **31.01.2024**
(21) Anmeldenummer: 20768000.0
(22) Anmeldetag: 03.09.2020
(51) Int. Cl.: B22F 10/00, A61F 2/30, A61L 27/12, A61L 27/32, B22F 7/00, B33Y 80/00, A61L 27/56

(54) **KNOCHENIMPLANTAT MIT BESCHICHTETER PORÖSER STRUKTUR**
BONE IMPLANT WITH COATED POROUS STRUCTURE
IMPLANT OSSEUX À STRUCTURE POREUSE REVÊTUE

(30) Priorität: 05.09.2019 EP 19195580
(43) Veröffentlichungstag der Anmeldung: 13.07.2022
(73) Patentinhaber: Waldemar Link GmbH & Co. KG, 22339 Hamburg (DE)
(72) Erfinder: LINK, Helmut D., 22397 Hamburg (DE); KÖNIG, Nicolas, 22846 Nordersted (DE)
(74) Vertreter: Glawe, Delfs, Moll
(86) Internationale Anmeldenummer: PCT/EP2020/074535
(87) Internationale Veröffentlichungsnummer: WO 2021/043867

(56) Entgegenhaltungen:
- WO-A2-02/085250
- RU-C1- 2 684 617
- RU-C1- 2 693 468
- US-A1- 2018 193 152
- US-B2- 9 456 901

## Beschreibung

Die Erfindung betrifft ein Knochenimplantat mit einem Hauptkörper, der in seinem Außenbereich eine offenzellige poröse Gitterstruktur aufweist, die mit einer knochenwachstumsfördernden Beschichtung umfassend Kalziumphosphat versehen ist.

Bei Implantaten, insbesondere in bzw. an Knochen zu implantierenden Endoprothesen sowie Augmentaten, kommt es besonders auf eine physiologisch günstige und stabile Verbindung zwischen Implantat und Knochen an. Ferner ist gewünscht, dass die Verbindung möglichst schnell hergestellt wird, um eine zügige Remobilisierung des Patienten zu erreichen. Zu diesem Zweck ist es bekannt, das Implantat mit einer knochenwachstumsfördernden Beschichtung zu versehen. Dies begünstigt das Wachstum von Knochenzellen und beschleunigt somit das Einwachsen des Implantats in bzw. am Knochen. Hierbei ist es im Grunde unerheblich, ob die Beschichtung auf glatte Oberflächen oder auf strukturierte Oberflächen aufgebracht wird. In beiden Fällen erfüllt sie ihre Bestimmung.

Für die Beschichtung sind verschiedene Materialien bekannt. Ihnen gemeinsam ist, dass sie bioaktiv sind und insbesondere knochenwachstumsfördernde Eigenschaften aufweisen. Sie können je nach Material auf verschiedene Weise aufgebracht werden, beispielsweise durch Plasmaspritzen, Sputtern oder durch Tauchbäder. Ein bereits seit den 1990er Jahren bekanntes Material mit günstigen knochenwachstumsfördernden Eigenschaften ist Kalziumphosphat (CaP). Insbesondere für die klinische Anwendung von dünnen und löslichen Beschichtungen ist es in der Implantologie etabliert.

In neuerer Zeit sind Implantate bekannt geworden, die durch additive Verfahren (beispielsweise in 3-D-Drucktechnik) hergestellt sind, womit sich eine regelmäßige, makroporöse Struktur erzeugen lässt (bspw. US 2018/193152 A1). Dies ist auch als trabekuläre Struktur in der Fachwelt bekannt. Diese besitzt bereits von Haus aus ein günstiges Einwachsverhalten. Von einer Beschichtung mit dem bekannten Kalziumphosphat konnte diese Struktur nur eingeschränkt profitieren. Es ist eine Sonderform bekannt geworden, bei der als Beschichtung Kalziumphosphat mit einem Kalzium/Phosphat-Verhältnis von 1,1 verwendet ist, wobei ein Bruschit-Anteil von mindestens 70% und ein Hydroxylapatit-Anteil bis zu 30% vorgesehen sind.

Weiter ist es zur Verbesserung des Einwachsverhaltens bekannt, Beschichtungen mit Anteil an Germanium zu versehen, die einen geringen Anteil von Kalziumphosphat im Bereich von 1 bis 15 Gewichtsprozent aufweisen (RU 2 684 617 C1).

Der Erfindung liegt die Aufgabe zugrunde, ein Knochenimplantat der eingangs genannten Art zu schaffen, das ein verbessertes Einwachsverhalten aufweist.

Die erfindungsgemäße Lösung liegt in den Merkmalen der unabhängigen Ansprüche 1 und 12 Vorteilhafte Weiterbildungen sind Gegenstand der abhängigen Ansprüche.

Bei einem Knochenimplantat mit einem Hauptkörper, der in seinem Außenbereich eine offenzellige poröse Gitterstruktur aufweist, die aus einer Vielzahl von regelmäßig angeordneten Elementarzellen gebildet ist, wobei die Elementarzellen als gebaute Struktur ausgeführt sind und jeweils aus einem Innenraum und einer Mehrzahl von den Innenraum umgebenden, miteinander verbundenen Stegen aufgebaut sind, wobei die poröse Gitterstruktur mit einer knochenwachstumsfördernden Beschichtung umfassend Kalziumphosphat versehen ist, ist erfindungsgemäß vorgesehen, dass die Kalziumphosphat-Beschichtung einen Hydroxylapatit-Anteil von höchstens 1 Gewichts-%, vorzugsweise weniger als 1 Gewichts-%, aufweist und eine in die Tiefe der porösen Gitterstruktur reichende Poreninnenbeschichtung bildet, wobei die Kalziumphosphat-Beschichtung eine Kristallphase aufweist, die Bruschit und Monetit umfasst und die mindestens 90 Gewichts-% beträgt, wobei der Bruschit-Anteil nicht weniger als 65 Gewichts-% ist.

Nachfolgend seien zuerst einige verwendete Begriffe erläutert:
Unter einer "offenzelligen" porösen Gitterstruktur wird verstanden, dass die Poren nicht jeweils für sich gesondert stehen, sondern dass die einzelnen Poren miteinander verbunden sind. Es ergibt sich somit insgesamt eine offene Zellstruktur, wobei die Poren in den einzelnen Zellen gebildet sind.

Unter einer "gebauten Struktur" wird verstanden, dass die Struktur additiv hergestellt ist. In Betracht kommen hier verschiedene Verfahren für die additive Herstellung. Besonders geeignet sind 3-D-Druckverfahren, wie beispielsweise Elektronenstrahlschmelzen bzw. selektives Laserschmelzen.

Unter in die Tiefe der porösen Gitterstruktur reichend wird hierbei verstanden, dass die Innenbeschichtung der Poren nicht nur in die an der Oberfläche liegenden Poren reicht, sondern auch die tiefer im Material liegenden (also oberflächenfernen) Poren erfasst, insbesondere auch solche Poren, die mehrere (Poren-)Lagen von der Oberfläche entfernt im Material liegen.

Unter einer "Wurzitstruktur" wird eine Struktur verstanden, die nach dem Vorbild der Wurzitkristallform gebildet ist (ähnlich wie unter einer Diamantstruktur eine Struktur nach dem Vorbild der Diamantkristallform verstanden wird).

Kern der Erfindung ist der Gedanke, die an sich bekannte Kalziumphosphat-Beschichtung dahingehend zu modifizieren und zu verbessern, dass sie praktisch frei ist von Hydroxylapatit (maximal 1 Gewichts-% bzw. vorzugsweise sogar weniger als 1 Gewichts-%). Typischerweise weisen aus dem Stand der Technik bekannte Kalziumphosphat-Beschichtungen einen Hydroxylapatit-Anteil von gut 20 Gewichts-% oder mehr auf, wie auch die eingangs erwähnte Sonderform. Indem erfindungsgemäß in dem Kalziumphosphat für die Beschichtung praktisch kein Hydroxylapatit mehr enthalten ist, kann, wie die Erfindung erkannt hat, eine höhere Löslichkeit erreicht werden. Diesen Vorteil nutzt die Erfindung in einem zweiten Schritt dazu, um die durch die Elementarzellen geschaffenen Hohlräume auch in ihrer Tiefe mit der Beschichtung zu versehen, und so die hohe Löslichkeit in größerem Umfang nutzen zu können.

Die Erfindung schafft so eine besondere Variante der Beschichtung und löst sich somit von dem bisher vorherrschenden Gedanken einer verhältnismäßig einfach aufzubringenden und gut haftenden Beschichtung an der Oberfläche des Implantats. Die Erfindung gibt durch den extrem niedrigen bzw. fehlenden Hydroxylapatitanteil die an sich gute Haftfähigkeit des üblicherweise für Implantatbeschichtungen verwendeten Kalziumphosphat-Materials auf. Auf den ersten Blick mag dies widersinnig erscheinen, jedoch hat die Erfindung erkannt, dass aus dem scheinbaren Nachteil der verminderten Haftfestigkeit ein entscheidender Vorteil realisiert werden kann. Es wird dadurch nämlich die Möglichkeit eröffnet, dass dieses an sich schlechter haftende Kalziumphosphat tiefer in die offenzellige Struktur eingebracht werden kann, und somit die knochenwachstumsfördernden Eigenschaften auch in der Tiefe der offenzelligen Struktur nutzbar gemacht werden. Überraschenderweise ergibt sich so ein ausgesprochen positiver Effekt, nämlich in Bezug auf ein schnelleres Einwachsen von Knochenzellen in das Implantat, und zwar ohne Verlust an guter Langzeitwirkung. Dies ist im Stand der Technik ohne Beispiel.

Außerdem ist die Kalziumphosphat-Beschichtung so beschaffen, dass sie eine Bruschit und Monetit umfassende Kristallphase aufweist. Diese kombinierte Kristallphase beträgt mindestens 90 Gewichts-%, vorzugsweise mindestens 95 Gewichts-%. Der erfindungsgemäß äußerst kleine Anteil an Hydroxylapatit erlaubt es, dass die kombinierte Kristallphase aus Bruschit/Monetit einen sehr hohen Anteil aufweisen kann, der ggf. sogar auch 99% oder mehr betragen kann. Hierbei ist vorgesehen, dass der Bruschit-Anteil nicht weniger als 65 Gewichts-% beträgt. Für den Monetit-Anteil gibt es keine untere Grenze. Da Bruschit besser von dem das Knochenimplantat aufnehmenden Organismus abgebaut wird, kann insbesondere durch den Mindestgehalt an Bruschit somit ein Optimum an Löslichkeit und somit knocheneinwachsendem Effekt gewährleistet werden.

Zweckmäßigerweise ist eine mittlere Dicke der Kalziumphosphat-Beschichtung so bemessen, dass die Innenräume der Elementarzellen miteinander verbunden bleiben, vorzugsweise zwischen 10 und 25 µm, weiter vorzugsweise 15µm +/- 5µm beträgt. Somit bleibt die offenzellige Struktur trotz der Beschichtung erhalten, was das Eindringen von Osteoblasten in die Zellen begünstigt. Das Osteointegrationsverhalten verbessert sich dadurch. Weiter weist die Kalziumphosphat-Beschichtung vorzugsweise ein Kalzium/Phosphat-Verhältnis im Bereich von 1,0 bis 1,2, vorzugsweise 1,05 bis 1,15, auf. Der höhere Phosphatanteil (verglichen mit dem Stand der Technik üblichen Verhältnis von etwa 1,6) sorgt hierbei für eine größere Löslichkeit, was wiederum, insbesondere zusammen mit der Beschichtung in der Tiefe der Struktur, das Knocheneinwachsverhalten fördert. Ferner weist die Kalziumphosphat-Beschichtung eine Bruschit-Phase auf, die mindestens 90 Gewichts-%, vorzugsweise mindestens 95 Gewichts-% (und ggf. bis zu 100 Gewichts-%), beträgt. Mit diesem Verhältnis lässt sich ein hoher Bruschit-Gehalt in dem Kalziumphosphat realisieren, und zwar bei einem Minimum von bzw. keinem Hydroxylapatit, mit den oben beschriebenen vorteilhaften Wirkungen. Insbesondere lässt sich so erreichen, dass die Kalziumphosphat-Beschichtung allseitig auf die gebaute Struktur der Elementarzellen, insbesondere ihren Stegen, aufgebracht ist. Insbesondere kann eine omnidirektionale (Rundum-) Auskleidung der Poren mit der Kalziumphosphat-Beschichtung gebildet werden, und zwar auch bei Poren, die komplexe bzw. hinterschnittene Strukturen aufweisen.

Weiter ist zweckmäßigerweise vorgesehen, dass die Kalziumphosphat-Beschichtung ungetempert ist. Dies vereinfacht zum einen die Herstellung und hat zum anderen den Vorteil, dass eine unerwünschte Verringerung des Bruschit-Anteils, wie sie durch die Temperung bewirkt würde, vermieden werden kann.

Mit Vorteil sind die Elementarzellen in Schichten angeordnet, wobei mehrere Schichten übereinander angeordnet sind, vorzugsweise als offenzellige trabekuläre Struktur. Es kann so eine tiefer reichende offenzellige Struktur geschaffen werden, die somit auch ein tieferes Einwachsen durch Osteointegration ermöglicht. Zweckmäßigerweise ist hierbei die Kalziumphosphat-Beschichtung auch in eine *tiefere* Schicht, vorzugsweise in allen Schichten, aufgebracht. Unter einer tieferen Schicht wird hierbei eine Schicht verstanden, die nicht unmittelbar an der Oberfläche liegt, sondern tiefer im Material. Die Verbindung zwischen Implantat und umgebenden Knochen wird dadurch weiter verbessert. Kurz- wie auch Langzeitbefestigungssicherheit werden dadurch begünstigt.

Zweckmäßigerweise ist die offenzellige poröse Gitterstruktur als 3D-Druck ausgeführt, vorzugsweise mittels Elektronenstrahlschmelzen (Electron Beam Melting - EBM) oder selektivem Laserschmelzen (Selective Laser Melting - SLM ). Auf diese Weise können rationell Komponenten rasch und kontrolliert aus metallischen Materialien hergestellt werden, und zwar gerade auch solche, die Strukturen mit komplexen und zahlreichen Hinterschnitten und Hohlräumen aufweisen. Die Struktur der Elementarzellen kann hierbei genau definiert werden. Dies ermöglicht eine definierte Anordnung der Zellen und der sie bildenden Elemente, insbesondere deren Stege. Insbesondere eignen sich diese Verfahren zur Herstellung des Implantats aus biokompatiblem Material, insbesondere metallischem Material, ausgewählt aus einer Gruppe umfassend Reintitan, Titanlegierungen, Kobaltchrom, Tantal, Edelstahl und Zirkonium, vorzugsweise aus Titan Grade 2 oder 4.

Mit Vorteil besteht der Hauptkörper aus demselben Material wie die offenzellige poröse Gitterstruktur. Somit kann dasselbe günstige, biokompatible Material auch für den Hauptkörper verwendet werden. Ferner ermöglicht dies einen nahtlosen und gegebenenfalls stufenlosen Übergang zwischen der offenzelligen porösen Struktur und dem eigentlichen Hauptkörper. Außerdem ist so eine rationellere Herstellung ermöglicht. Das gilt vor allem dann, wenn auch der Hauptkörper einen tragenden Bereich (Tragbereich) aufweist. Zweckmäßigerweise kann dieser ebenfalls eine gewisse Porosität aufweisen, die aber typischerweise abweicht von der offenzelligen Gitterstruktur und vorzugsweise geringer ist. Besonders zweckmäßig ist es, wenn der Tragbereich aus massivem Material ausgeführt ist. Dies ergibt nicht nur eine höhere mechanische Festigkeit, sondern damit kann auch eine Sperrwirkung erzielt werden im Sinne einer Schottwand, beispielsweise um innere und äußere Bereiche abzugrenzen oder um einen Durchtritt von Materialien, wie Knochenzement und/oder Körperflüssigkeiten, zu verhindern.

Besonders zweckmäßig ist es, wenn der Tragbereich unitär mit der offenzelligen porösen Struktur ausgeführt sein kann. Dies ermöglicht eine besonders rationelle Herstellung und einen stufenlosen Übergang. Gerade letzteres bietet den Vorteil geringster Irritation vom umgebenden Gewebe und begünstigt somit weiter das Einwachsverhalten.

Vorzugsweise sind die Elementarzellen in einer Wurzitstruktur ausgeführt. Diese unterscheidet sich von der bekannten Diamantstruktur darin, dass die Diamantstruktur in allen drei Dimensionen des Raums dieselbe Steifigkeit aufweist, während die Wurzitstruktur unterschiedliche Steifigkeit in den Raumrichtungen aufweist. Dies ermöglicht eine bessere Anpassung des Steifigkeitsverhaltens mittels der Wurzitstruktur an die anatomischen Verhältnisse, und erhöht so im Ergebnis die Biokompatibilität des Implantats.

Mit Vorteil sind die Elementarzellen makroporös ausgeführt. Darunter wird vorliegend insbesondere verstanden, dass sie mit ihren Innenräumen Makroporen bilden, deren Weite im Bereich zwischen 0,4 und 2 mm, vorzugsweise 0,7 bis 1,5 mm, liegt. Die Tiefe der porösen Struktur ist dabei zweckmäßigerweise so gewählt, dass mindestens zwei Schichten von Elementarzellen übereinander liegen. Eine solche makroporöse offenzellige Struktur bietet mit großen verbundenen Freiräumen besonders günstige Bedingungen für das vernetzte Einwachsen von Knochen.

Vorzugsweise ist im Gegensatz zur verhältnismäßig großen Ausführung der von den Elementarzellen gebildeten Poren die Beschichtung vergleichsweise dünn ausgeführt. Zweckmäßigerweise weist die Beschichtung eine Dicke von nur zwischen 10 und 20 µm auf. Mit einer solchen dünnen Beschichtung kann eine Art Innenauskleidung der von den Elementarzellen gebildeten Makroporen erreicht werden, und zwar derart, dass die Porosität und insbesondere die offenzellige Struktur (d. h. die Verbindung zwischen den einzelnen Freiräumen) einwandfrei erhalten bleiben. Dies ist besonders günstig in Bezug auf das Knocheneinwachsverhalten, und zwar sowohl für Osteoinduktion sowie auch für Osteokonduktion. Zweckmäßigerweise ist das Verhältnis zwischen der Weite des Freiraums der Elementarzellen einerseits und der Dicke der Beschichtung andererseits so gewählt, dass die Weite des Freiraums mindestens das Zehnfache, vorzugsweise zwischen dem 30-fachen und dem 200-fachen, der Dicke der Beschichtung beträgt.

Die Erfindung erstreckt sich ferner auf ein Verfahren zur Herstellung eines entsprechend beschichteten Implantats mit einem Hauptkörper, der in seinem Außenbereich eine offenzellige poröse Gitterstruktur aufweist, die aus einer Vielzahl von regelmäßig angeordneten Elementarzellen gebildet ist, mit den Schritten: Aufbauen der regelmäßig angeordneten Elementarzellen als gebaute Struktur aus jeweils einem Innenraum und einer Mehrzahl von den Innenraum umgebenden, miteinander verbundenen Stegen derart, dass die Innenräume miteinander verbunden sind, Beschichten der porösen Gitterstruktur mit einer knochenwachstumsfördernden Beschichtung umfassend Kalziumphosphat, wobei erfindungsgemäß vorgesehen ist, dass beim Beschichten die Beschichtung hergestellt wird mit einem Hydroxylapatit-Anteil von höchstens 1 Gewichts-% und bis in die Tiefe der porösen Gitterstruktur als Poreninnenbeschichtung aufgetragen wird. Die Beschichtung wird so durchgeführt, dass sie eine Kristallphase aufweist, die Bruschit und Monetit umfasst und die mindestens 90 Gewichts-%, vorzugsweise mindestens 95 Gewichts-%, beträgt, wobei der Bruschit-Anteil nicht weniger als 65 Gewichts-% ist. Zur näheren Erläuterung wird auf vorstehende Beschreibung verwiesen.

Vorzugsweise wird die Beschichtung allseitig auf die poröse Gitterstruktur als Ausfällung aufgetragen, vorzugsweise mittels elektrochemischen Verfahrens. Damit kann eine omnidirektionale Poreninnenbeschichtung, d.h. Auskleidung der Poren mit der knochenwachstumsfördernden Beschichtung erreicht werden. Mit Vorteil wird für das elektrochemische Verfahren ein Strom verwendet, der einer Stromkurve folgt, die nach einem anfänglichen Spitzenstrom auf einen niedrigeren Arbeitsstrom zurückfällt. Die Erfindung hat erkannt, dass dieser verringerte Strom zu einer verbesserten Ausfällungsreaktion insbesondere der kombinierten Kristallphase mit Bruschit/Monetit des Kalziumphosphats auf den Strukturelementen der Elementarzellen führt, und zwar besonders in der Tiefe der Struktur. Ferner lässt sich damit zuverlässig eine gleichmäßige dünne Beschichtung erzielen.

Vorzugsweise wird ferner auf eine anschließende Temperung nach der elektrochemischen Bearbeitung verzichtet. Damit kann eine unerwünschte Kristallumwandlung vermieden werden, sodass die Bruschit-Phase des Kalziumphosphats weiterhin den gewünschten hohen Anteil behält.

Für weitere vorteilhafte Gestaltungen und zur näheren Beschreibung wird im Übrigen auf vorstehende Erläuterung zu dem Implantat verwiesen, die entsprechend auch für das Verfahren gilt.

Zusammenfassend ist festzustellen, dass mit der Beschichtung gemäß der Erfindung ein verbessertes Knocheneinwachsen erreicht werden kann, wie Versuche belegt haben.

Die Erfindung wird nachfolgend unter Bezugnahme auf die beigefügte Zeichnung anhand vorteilhafter Ausführungsbeispiele näher erläutert. Es zeigen:
- Fig. 1: ein Ausführungsbeispiel für ein Implantat gemäß einem Ausführungsbeispiel der Erfindung;
- Fig. 2: eine Detailansicht einer Elementarzelle der porösen Struktur des Implantats gemäß Figur 1;
- Fig. 3: eine schematische Ansicht der Elementarzellen und der sie formenden Elemente;
- Fig. 4a, b: Schnittansichten in zwei orthogonalen Richtungen der porösen Struktur;
- Fig. 5: eine Abbildung eines Augmentats gemäß einem zweiten Ausführungsbeispiels der Erfindung;
- Fig. 6a, b: eine schematische Seitenansicht sowie Frontalansicht des Augmentats gemäß Figur 5;
- Fig. 7: eine Vergleichstabelle darstellend das Knocheneinwachsverhalten; und
- Fig. 8: ein Diagramm zum Stromverlauf beim elektrochemischen Beschichten gemäß der Erfindung.

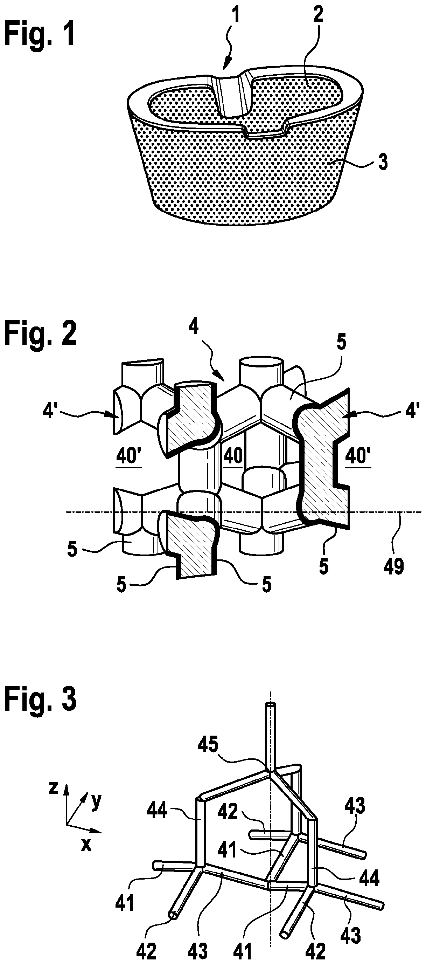

Ein erstes Ausführungsbeispiel für ein erfindungsgemäßes Implantat ist in Figur 1 dargestellt. Es handelt sich hierbei um einen Konus 1 für die tibiale Komponente einer Kniegelenkendoprothese (nicht dargestellt).

Der Konus 1 bildet einen Ersatz für defektes Knochenmaterial am proximalen Ende der Tibia, um so Hohlräume zu füllen, die durch fehlendes schadhaftes Knochenmaterial entstanden sind. Auf diese Weise wird eine vollständige Basis geschaffen, auf der die tibiale Komponente der Kniegelenkendoprothese sicher angeordnet werden kann. Zu diesem Zweck ist der Konus 1 hergestellt unter Verwendung der offenzelligen porösen Gitterstruktur, die mit einer erfindungsgemäßen Beschichtung zur Verbesserung des Anwachsens bzw. Einwachsens von Knochenmaterial versehen ist. Hierbei ist auf einem Hauptkörper 2 die offenzellige poröse Gitterstruktur 3 aufgebracht.

Insbesondere dank der Anordnung dieser offenzelligen porösen Gitterstruktur 3 auf der Außenseite des Konus 1 kann so ein gutes Einwachsverhalten von Knochenmaterial des umgebenden Tibia-Knochens (nicht dargestellt) erreicht werden, wodurch sich eine schnelle und sichere Befestigung des Konus 1 in der Tibia ergibt.

Die poröse Struktur 3 ist gebildet durch eine Vielzahl von regelmäßig angeordneten Elementarzellen 4. Eine Detailansicht einer Elementarzelle 4 und ihrer Einbindung in umgebende Elementarzellen ist in Figur 2 dargestellt. Die Elementarzelle 4 weist einen Innenraum 40 auf, der mit dem Innenraum 40` benachbarter Elementarzellen 4' verbunden ist. Die Elementarzellen sind regelmäßig entlang einer Schichtungsebene 49 angeordnet. Vorteilhafterweise sind mehrere Schichtungsebenen übereinander angeordnet.

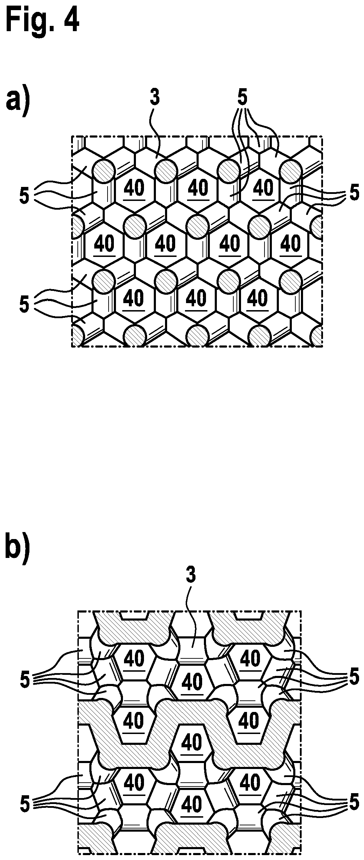

Die regelmäßige Anordnung der Elementarzellen ist insbesondere aus den Seitenansichten in Figur 4a, b gut ersichtlich. Sie zeigen isometrische Ansichten entlang der beiden orthogonalen Achsen (s. Achsen x, y in Fig. 3), welche die Schichtungsebene 49 definieren. Man erkennt, dass sich in den beiden Richtungen verschiedene Querschnittsansichten ergeben, insbesondere in Bezug auf die Gestaltung des Innenraums 40. Dies ist eine spezielle Eigenschaft der verwendeten Kristallstruktur, nämlich der Wurzitstruktur. Sie sorgt dafür, dass die so gebildete offenzellige poröse Gitterstruktur unterschiedliche Kompressions-Steifigkeiten in verschiedenen Richtungen des Raumes aufweist, was günstig ist in Bezug auf eine Anpassung an die anatomischen Verhältnisse des Knochens. Man erkennt dort ferner, dass benachbarte Innenräume 40 miteinander verbunden sind, so dass die durch die Elementarzellen 4 mit ihren Innenräumen 40 gebildeten Makroporen offenzellig miteinander verbunden sind (sie bilden sog. "inter-connected pores").

Der eigentliche Aufbau der Elementarzellen 4 ist schematisch in Figur 3 dargestellt. In dem dargestellten Ausführungsbeispiel sind die Elementarzellen 4 geformt aus Grundelementen 45, die jeweils als Tetrapoden ausgeführt sind. Es versteht sich, dass auch andere Grundelemente als Tetrapoden vorgesehen sein können. Jeder dieser Tetrapoden weist vier als Stege ausgeführte Beine 41, 42, 43, 44 auf, die jeweils an einem Ende miteinander verbunden sind und so einen Knotenpunkt bilden. Die Tetrapoden können regulär oder irregulär gebildet sein mit gleichmäßigen Beinlängen oder verschiedenen Beinlängen. Dargestellt ist eine regelmäßige Ausführungsform, bei der die Beine gleich lang sind und jedes Bein denselben Winkel mit jedem der anderen Beine einschließt. Bei der Anordnung der Tetrapoden in einer ebenen Schichtung sind jeweils drei Beine 41, 42, 43 aufstehend auf eine Ebene angeordnet, während das vierte Bein 44 senkrecht zu der Ebene orientiert ist. Dieses vierte Bein stellt somit eine Verbindung zu den Tetrapoden einer darüber angeordneten Schichtungsebene dar (siehe Figur 3).

Durch die Wahl der Anzahl der Schichtungsebenen kann die Tiefe der offenzelligen porösen Struktur gesteuert werden. So können bspw. drei oder vier oder fünf übereinanderliegende Schichten vorgesehen sein (siehe Figur 4a, b), typischerweise sind mindestens jedoch zwei übereinanderliegende Schichten vorgesehen. Als Material für die offenzellige poröse Struktur wird vorzugsweise eine Titanlegierung oder Reintitan verwendet.

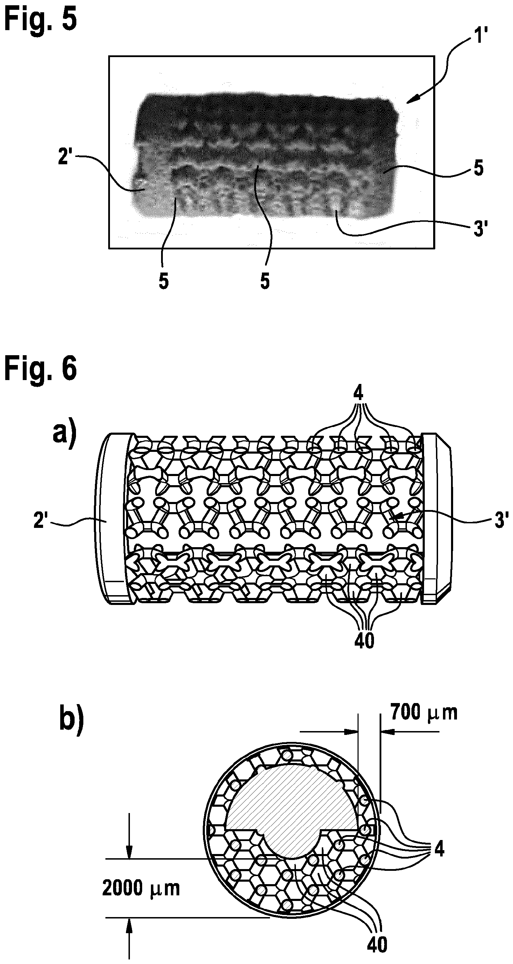

Ein zweites Ausführungsbeispiel ist in den Figuren 5 und 6 dargestellt. Bei Figur 5 handelt es sich um eine fotografische Abbildung. Sie zeigt ein zylindrisches Augmentat 1', wie es ebenfalls zum Auffüllen von Knochendefekten oder gegebenenfalls auch zu Zwecken einer Fusion von benachbarten Knochenelementen, insbesondere Wirbelkörpern, exemplarisch verwendet werden kann. Es weist einen im Wesentlichen als Hülse ausgebildeten Hauptkörper 2' auf, der von einer generell zylinderförmigen Gestalt ist. Auf seiner Mantelfläche ist der Hauptkörper 2' mit der offenzelligen porösen Gitterstruktur 3` versehen. Sie ist, wie insbesondere in der schematischen Ansicht in Figur 6a, b gut zu erkennen ist, ebenfalls aus Elementarzellen 4 mit ihren miteinander verbundenen Innenräumen 40 gebildet, wobei die Elementarzellen 4 wiederum aus Tetrapoden als Grundelementen 45 bestehen.

Wie insbesondere aus der fotografischen Abbildung in Figur 5 gut zu erkennen ist, ist die durch die Elementarzellen 4 gebildete offenzellige poröse Gitterstruktur 3' mit einer in der Abbildung etwas rau wirkenden Beschichtung 5 versehen. Die Beschichtung 5 ist flächig sowohl auf der offenzelligen porösen Gitterstruktur 3` wie auch an den beiden Endbereichen des Hauptkörpers 2' und weiter auch in der Tiefe der Struktur 3' in den Innenräumen 40 der Elementarzellen 4 aufgebracht.

Beispielhafte Abmessungen für die Länge und Breite des zylinderhülsenartigen Hauptkörpers 2' sind 12 mm Länge bzw. 6 mm Durchmesser als Breite. Hierbei weisen die Innenräume 40 der die offenzellige poröse Struktur 3' bildenden Elementarzellen 4 eine Weite von etwa 700 µm auf, und die Tiefe der offenzelligen porösen Struktur 3' erstreckt sich über etwa 2000 µm. In Elementarzellen 4 betrachtet resultiert daraus eine Tiefe von knapp drei Schichten von Elementarzellen 4.

Die Beschichtung 5 weist eine kombinierte Kristallphase von Bruschit und Monetit mit einem Anteil von 95 Gewichts-% auf, Wobei der Anteil von Bruschit mindestens 65 Gewichts-% beträgt. Ferner kleidet die Beschichtung 5 die Elementarzellen 4 mit ihren Hohlräumen 40 vollständig aus, und zwar nicht nur in der obersten Schicht, sondern auch in den darunterliegenden Schichten.

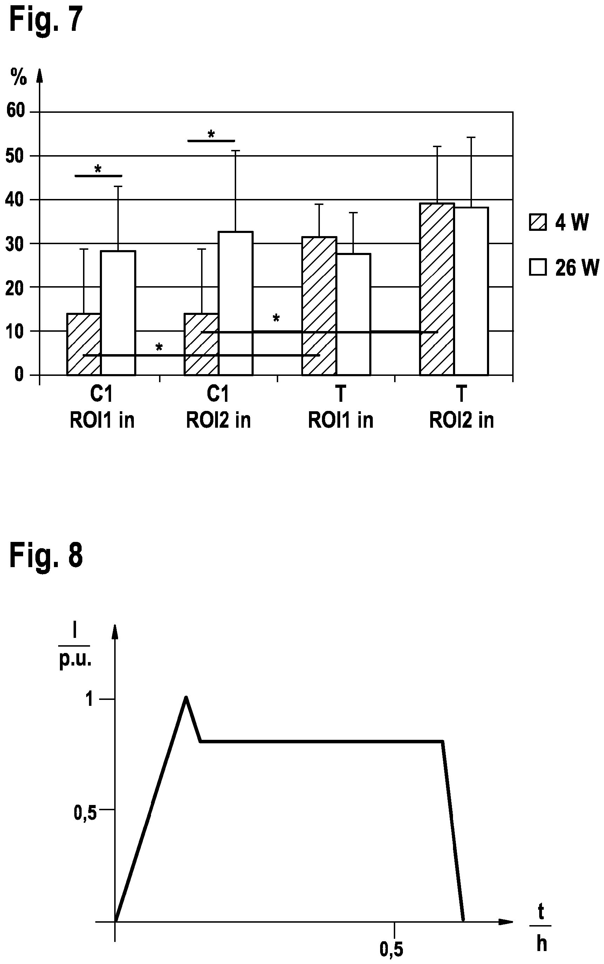

Daraus ergibt sich erfindungsgemäß ein deutlich verbessertes Einwachsen von Knochenmaterial im Rahmen der Osteointegration und Osteokonduktion. Ergebnisse für einen Vergleichsversuch mit einem Vergleichs-Implantat, dass eine gleichgestaltete offenzellige poröser Struktur aufweist, jedoch ohne erfindungsgemäße Beschichtung 5, sind in Figur 7 dargestellt. Dort ist eine quantitative histomorphometrische Analyse gezeigt, wobei entlang der Y-Achse das prozentual ausgedrückte Knochen/Implantat-Kontaktverhältnis dargestellt ist, und zwar für zwei verschiedene Gebiete (ROI1 und ROI2). Die beiden linken Säulenpaare stehen für das Vergleichs-Implantat ("C1"), und die beiden rechten Säulenpaare stehen für das getestete erfindungsgemäße Implantat ("T"). Die linke Säule in jedem Säulenpaar zeigt das kurzfristige Einwachsverhalten (gemessen nach 4 Wochen) und die rechte Säule in jedem Säulenpaar zeigt das langfristige Einwachsverhalten (gemessen nach 26 Wochen). Man erkennt deutlich, dass bei dem erfindungsgemäßen Implantat ("T") bereits nach 4 Wochen ein hervorragendes Einwachsen von Knochenmaterial erreicht ist, wobei das Vergleichsbeispiel einen ähnlichen Wert erst nach der gut sechsfachen Zeit, nämlich nach 26 Wochen erreicht. Dies belegt eindrucksvoll die Knochenwachstum fördernde Eigenschaft der erfindungsgemäßen Beschichtung.

Für die Beschichtung wird zweckmäßigerweise ein elektrochemisches Verfahren verwendet. Der Stromverlauf bei der elektrochemischen Beschichtung ist in Figur 8 dargestellt.

Man erkennt, dass zu Anfang ein hoher Spitzenstrom eingestellt wird, der dann reduziert wird auf einen niedrigeren Arbeitsstrom. Mit diesem Stromregime lässt sich eine besonders gute und für die dünne und gleichmäßige Beschichtung besonders geeignete Ausfällreaktion des Kalziumphosphats erreichen, wobei die kombinierte Bruschit/Monetit-Phase mit ihrem hohen Anteil von gut 95 % entsteht.

## Patentansprüche

1. Knochenimplantat mit einem Hauptkörper (2), der in seinem Außenbereich eine offenzellige poröse Gitterstruktur (3) aufweist, die aus einer Vielzahl von regelmäßig angeordneten Elementarzellen (4) gebildet ist, wobei die Elementarzellen (4) als gebaute Struktur ausgeführt sind und jeweils aus einem Innenraum (40) und einer Mehrzahl von den Innenraum (40) umgebenden, miteinander verbundenen Stegen (41, 42, 43, 44) aufgebaut sind, wobei die poröse Gitterstruktur (3) mit einer knochenwachstumsfördernden Beschichtung (5) umfassend Kalziumphosphat versehen ist,
**dadurch gekennzeichnet, dass**
die Kalziumphosphat-Beschichtung (5) einen Hydroxylapatit-Anteil von höchstens 1 Gewichts-% aufweist und eine in die Tiefe der porösen Gitterstruktur (3) reichende Poreninnenbeschichtung bildet, und
die Kalziumphosphat-Beschichtung (5) eine Kristallphase aufweist, die Bruschit und Monetit umfasst und die mindestens 90 Gewichts-% beträgt, wobei der Bruschit-Anteil nicht weniger als 65 Gewichts-% ist.

2. Knochenimplantat nach Anspruch 1, **dadurch gekennzeichnet, dass** die Kristallphase der Kalziumphosphat-Beschichtung (5) mindestens 95 Gewichts-% beträgt.

3. Knochenimplantat nach Anspruch 1 oder 2, **dadurch gekennzeichnet, dass** die Kalziumphosphat-Beschichtung (5) ein Kalzium/Phosphat-Verhältnis im Bereich von 1,0 bis 1,2, vorzugsweise 1,05 bis 1,15, aufweist.

4. Knochenimplantat nach einem der vorangehenden Ansprüche, **dadurch gekennzeichnet, dass** eine mittlere Dicke der Kalziumphosphat-Beschichtung (5) so bemessen ist, dass die Innenräume der Elementarzellen (4) miteinander verbunden bleiben, vorzugsweise zwischen 10 und 25 µm weiter vorzugsweise 15µm +/- 5µm beträgt.

5. Knochenimplantat nach einem der vorangehenden Ansprüche, **dadurch gekennzeichnet, dass** die Kalziumphosphat-Beschichtung (5) ungetempert ist.

6. Knochenimplantat nach einem der vorangehenden Ansprüche, **dadurch gekennzeichnet, dass** die Kalziumphosphat-Beschichtung (5) allseitig auf die gebaute Struktur der Elementarzellen (4), insbesondere ihren Stegen (41, 42, 43, 44), aufgebracht ist, vorzugsweise eine omnidirektionale Poren-Innenbeschichtung gebildet ist.

7. Knochenimplantat nach einem der vorangehenden Ansprüche, **dadurch gekennzeichnet, dass** die Elementarzellen (4) in Schichten angeordnet sind, wobei mehrere Schichten übereinander angeordnet sind, vorzugsweise als offenzellige trabekuläre Struktur, und/oder die Elementarzellen (4) in einer Wurzitstruktur ausgeführt sind.

8. Knochenimplantat nach einem der vorangehenden Ansprüche, **dadurch gekennzeichnet, dass** die offenzellige poröse Gitterstruktur (3) als 3D-Druck ausgeführt ist, vorzugsweise mittels Elektronenstrahlschmelzen oder selektivem Laserschmelzen

9. Knochenimplantat nach einem der vorangehenden Ansprüche, **dadurch gekennzeichnet, dass** der Hauptkörper (2) aus demselben Material wie die offenzellige poröse Gitterstruktur besteht.

10. Knochenimplantat nach einem der vorangehenden Ansprüche, **dadurch gekennzeichnet, dass** der Hauptkörper (2) einen Tragbereich aufweist, dessen Porosität geringer ist als eine Porosität der offenzelligen porösen Gitterstruktur (3) , wobei der Tragbereich vorzugsweise massiv ausgeführt ist, und/oder der Tragbereich und die offenzellige poröse Gitterstruktur unitär ausgeführt sind.

11. Knochenimplantat nach einem der vorangehenden Ansprüche, **dadurch gekennzeichnet, dass** die Innenräume (40) der Elementarzellen (4) Makroporen bilden, deren Weite mindestens das Zehnfache, vorzugsweise zwischen dem 30-fachen und dem 200-fachen, der Dicke der Beschichtung (5) beträgt, und/oder die Weite der Poren im Bereich zwischen 0,4 und 2 mm, vorzugsweise 0,7 bis 1,5 mm, liegt und die Beschichtung (5) eine Dicke zwischen 10 und 20 µm aufweist.

12. Verfahren zur Herstellung eines beschichteten Knochenimplantats mit einem Hauptkörper, der in seinem Außenbereich eine offenzellige poröse Gitterstruktur aufweist, die aus einer Vielzahl von regelmäßig angeordneten Elementarzellen gebildet ist, mit den Schritten:
Aufbauen der regelmäßig angeordneten Elementarzellen als gebaute Struktur aus jeweils einem Innenraum und einer Mehrzahl von den Innenraum umgebenden, miteinander verbundenen Stegen derart, dass die Innenräume miteinander verbunden sind,
Beschichten der porösen Gitterstruktur mit einer knochenwachstumsfördernden Beschichtung umfassend Kalziumphosphat,
**dadurch gekennzeichnet, dass**
die Beschichtung hergestellt wird mit einem Hydroxylapatit-Anteil von höchstens 1 Gewichts-% und bis in die Tiefe der porösen Gitterstruktur als Poreninnenbeschichtung aufgetragen wird, wobei die Beschichtung eine Kristallphase aufweist, die Bruschit und Monetit umfasst und die mindestens 90 Gewichts-% beträgt, wobei der Bruschit-Anteil nicht weniger als 65 Gewichts-% ist.

13. Verfahren nach Anspruch 12, **dadurch gekennzeichnet, dass** die Kristallphase der Beschichtung mindestens 95 Gewichts-% beträgt.

14. Verfahren nach Anspruch 12 oder 13, **dadurch gekennzeichnet, dass** die Beschichtung allseitig auf die poröse Gitterstruktur als Ausfällung aufgetragen wird, vorzugsweise mittels elektrochemischen Verfahrens.

15. Verfahren nach Anspruch 14, **dadurch gekennzeichnet, dass** für das elektrochemische Verfahren ein Strom verwendet wird, der einer Stromkurve folgt, die nach einem anfänglichen Spitzenstrom auf einen niedrigeren Arbeitsstrom zurückfällt.

## Claims

1. A bone implant, having a main body (2) with an open-cell porous lattice structure (3) in its outer region, said lattice structure being formed from a plurality of regularly arranged unit cells (4), wherein the unit cells (4) are embodied as an assembled structure and are constructed from an interior space (40) and a plurality of interconnected bars (41, 42, 43, 44) surrounding the interior space (40), wherein the porous lattice structure (3) is provided with a coating (5) which promotes bone growth, comprising calcium phosphate,
**characterized in that**
the calcium phosphate coating (5) has a hydroxyapatite content of less than or equal to 1 wt%, and forms an inner pore coating extending deep into the porous lattice structure (3), and
the calcium phosphate coating (5) has a crystal phase which comprises brushite and monetite, and which is at least 90 wt%, wherein the brushite fraction is not less than is 65 wt%.

2. The bone implant according to claim 1, **characterized in that** the crystal phase of the calcium phosphate coating (5) is at least 95 wt%.

3. The bone implant according to claim 1 or 2, **characterized in that** the calcium phosphate coating (5) has a calcium/phosphate ratio in the range from 1.0 to 1.2, preferably 1.05 to 1.15.

4. The bone implant according to any one of the preceding claims, **characterized in that** the average thickness of the calcium phosphate coating (5) is dimensioned such that the interior spaces of the unit cells (4) remain interconnected, preferably being between 10 and 25 pm, more preferably 15 µm ± 5 µm.

5. The bone implant according to any one of the preceding claims, **characterized in that** the calcium phosphate coating (5) is unannealed.

6. The bone implant according to any one of the preceding claims, **characterized in that** the calcium phosphate coating (5) is applied to all sides of the assembled structure of the unit cells (4), in particular its bars (41, 42, 43, 44), preferably with an omni-directional inner pore coating being formed.

7. The bone implant according to any one of the preceding claims, **characterized in that** the unit cells (4) are arranged in layers, with several layers arranged on top of one another, preferably as an open-cell trabecular structure, and/or the unit cells (4) are embodied in a wurtzite structure.

8. The bone implant according to any one of the preceding claims, **characterized in that** the open-cell porous lattice structure (3) is embodied as a 3D printed structure, preferably by means of electron beam melting or selective laser melting.

9. The bone implant according to any one of the preceding claims, **characterized in that** the main body (2) consists of the same material as the open-cell porous lattice structure.

10. The bone implant according to any one of the preceding claims, **characterized in that** the main body (2) has a supporting region, said supporting region having a lower porosity than the porosity of the open-cell porous lattice structure (3), the supporting preferably being embodied in a compact manner, and/or the supporting region and the open pore lattice structure being embodied in a unitary manner.

11. The bone implant according to any one of the preceding claims, **characterized in that** the interior spaces (40) of the unit cells (4) form macropores, the width of which is at least ten times, preferably between 30 times and 200 times, the thickness of the coating (5), or the width of the pores is in the range between 0.4 and 2 mm, preferably 0.7 to 1.5 mm, and the coating (5) has a thickness between 10 and 20 µm.

12. A method for producing a coated bone implant having a main body which has an open-cell, porous lattice structure in its outer region, said lattice structure being formed from a plurality of regularly arranged unit cells, the method including the steps of:
building the regularly arranged unit cells into an assembled structure, each consisting of an interior space and a plurality of interconnected bars surrounding the interior space in such a way that the interior spaces are connected to each other,
coating the porous lattice structure with a coating which promotes bone growth, comprising calcium phosphate,
**characterized in that**
the coating is produced with a hydroxyapatite content of less than or equal to 1 wt%, and is applied deep into the porous lattice structure as an inner pore coating, wherein the coating has a crystal phase which comprises brushite and monetite, and which is at least 90 wt%, wherein the brushite fraction is not less than is 65 wt%.

13. The method according to claim 12, **characterized in that** the crystal phase of the coating is at least 95 wt%.

14. The method according to claim 12 or 13, **characterized in that** the coating is applied to all sides of the porous lattice structure as a precipitate, preferably by means of an electrochemical method.

15. The method according to claim 14, **characterized in that** a current is used for the electrochemical method, which follows a current curve which, after an initial peak current, falls back to a lower working current.

## Revendications

1. Implant osseux avec un corps principal (2), qui présente dans sa zone extérieure une structure en grille (3) poreuse à cellules ouvertes, qui est formée d'une multitude de cellules élémentaires (4) disposées régulièrement, dans lequel les cellules élémentaires (4) sont conçues sous forme d'une structure bâtie et chacune constituée d'un intérieur (40) et d'une pluralité de passerelles (41, 42, 43, 44) reliées les unes aux autres entourant l'intérieur (40), dans lequel la structure en grille (3) poreuse est dotée d'un revêtement favorisant la croissance osseuse (5) comprenant du phosphate de calcium,
**caractérisé en ce que**
le revêtement de phosphate de calcium (5) présente une teneur en hydroxyapatite d'au plus 1 % en poids et forme un revêtement de pores internes qui s'étend dans la profondeur de la structure en grille (3) poreuse, et
le revêtement de phosphate de calcium (5) présente une phase cristalline qui comprend de la bruschite et de la monétite et qui représente au moins 90 % en poids, dans lequel la teneur en bruschite n'est pas inférieure à 65 % en poids.

2. Implant osseux selon la revendication 1, **caractérisé en ce que** la phase cristalline du revêtement de phosphate de calcium (5) représente au moins 95 % en poids.

3. Implant osseux selon la revendication 1 ou 2, **caractérisé en ce que** le revêtement de phosphate de calcium (5) présente un rapport calcium/phosphate dans la plage de 1,0 à 1,2, de préférence 1,05 à 1,15.

4. Implant osseux selon l'une quelconque des revendications précédentes, **caractérisé en ce qu'**une épaisseur moyenne du revêtement de phosphate de calcium (5) est dimensionnée de sorte que les intérieurs des cellules unitaires (4) restent reliés les uns aux autres, représentant de préférence entre 10 et 25 um, de manière davantage préférée 15 um +/- 5 µm.

5. Implant osseux selon l'une quelconque des revendications précédentes, **caractérisé en ce que** le revêtement de phosphate de calcium (5) n'est pas trempé.

6. Implant osseux selon l'une quelconque des revendications précédentes, **caractérisé en ce que** le revêtement de phosphate de calcium (5) est appliqué sur toutes les faces de la structure bâtie des cellules élémentaires (4), en particulier leurs passerelles (41, 42, 43, 44), un revêtement de pores internes omnidirectionnel est de préférence formé.

7. Implant osseux selon l'une quelconque des revendications précédentes, **caractérisé en ce que** les cellules élémentaires (4) sont disposées en couches, dans lequel plusieurs couches sont disposées les unes au-dessus des autres, de préférence sous forme de structure trabéculaire à cellules ouvertes, et/ou les cellules élémentaires (4) sont conçues dans une structure en wurtzite.

8. Implant osseux selon l'une quelconque des revendications précédentes, **caractérisé en ce que** la structure en grille (3) poreuse à cellules ouvertes est conçue sous forme d'impression 3D, de préférence au moyen d'une fusion par faisceau d'électrons ou d'une fusion sélective au laser.

9. Implant osseux selon l'une quelconque des revendications précédentes, **caractérisé en ce que** le corps principal (2) est constitué du même matériau que la structure en grille poreuse à cellules ouvertes.

10. Implant osseux selon l'une quelconque des revendications précédentes, **caractérisé en ce que** le corps principal (2) présente une zone de support dont la porosité est inférieure à une porosité de la structure en grille (3) poreuse à cellules ouvertes, dans lequel la zone de support est de préférence conçue sous forme de masse, et/ou la zone de support et la structure en grille poreuse à cellules ouvertes sont conçues de manière à être unitaires.

11. Implant osseux selon l'une quelconque des revendications précédentes, **caractérisé en ce que** les intérieurs (40) des cellules élémentaires (4) forment des macropores dont la largeur représente au moins dix fois, de préférence entre 30 fois et 200 fois, l'épaisseur du revêtement (5), et/ou la largeur des pores se situe dans la plage comprise entre 0,4 et 2 mm, de préférence 0,7 à 1,5 mm, et le revêtement (5) présente une épaisseur comprise entre 10 et 20 µm.

12. Procédé de fabrication d'un implant osseux avec un corps principal, qui présente dans sa zone extérieure une structure en grille poreue à cellules ouvertes, qui est formée d'une multitude de cellules élémentaires disposées régulièrement, avec les étapes :
de construction des cellules élémentaires disposées régulièrement sous forme d'une structure bâtie chacune constituée d'un intérieur et d'une pluralité de passerelles reliées les unes aux autres entourant l'intérieur, de sorte que les intérieurs soient reliés les uns aux autres,
de revêtement de la structure en grille poreuse avec un revêtement favorisant la croissance osseuse comprenant du phosphate de calcium,
**caractérisé en ce que**
le revêtement est fabriqué avec une teneur en hydroxyapatite d'au plus 1 % en poids et est appliqué jusque dans la profondeur de la structure en grille poreuse sous forme de revêtement de pores internes, dans lequel le revêtement présente une phase cristalline qui comprend de la bruschite et de la monétite et qui représente au moins 90 % en poids, dans lequel la teneur en bruschite n'est pas inférieure à 65 % en poids.

13. Procédé selon la revendication 12, **caractérisé en ce que** la phase cristalline du revêtement représente au moins 95 % en poids.

14. Procédé selon la revendication 12 ou 13, **caractérisé en ce que** le revêtement est appliqué sous forme de précipitation sur toutes les faces de la structure en grille poreuse, de préférence au moyen d'un procédé électrochimique.

15. Procédé selon la revendication 14, **caractérisé en ce qu'**un courant est utilisé pour le processus électrochimique, qui suit une courbe de courant qui retombe à un courant de travail inférieur après un courant de pointe initial.
